# EUROPEAN PATENT SPECIFICATION

(11) **EP 2 776 118 B1**
(45) Date of publication and mention of the grant of the patent: **22.09.2021**
(21) Application number: 12847854.2
(22) Date of filing: 08.11.2012
(51) Int. Cl.: A61N 1/05, A61N 1/36, A61N 1/375, A61N 1/04

(54) **MEDICAL DEVICE CONTACT ASSEMBLIES FOR USE WITH IMPLANTABLE LEADS**
KONTAKTANORDNUNGEN FÜR EINE MEDIZINISCHE VORRICHTUNG ZUR VERWENDUNG MIT IMPLANTIERBAREN LEITUNGEN
DISPOSITIFS MÉDICAUX ET ENSEMBLES DE CONTACT POUR L'UTILISATION AVEC DES DÉRIVATIONS IMPLANTABLES

(30) Priority: 08.11.2011 US 201113291985
(43) Date of publication of application: 17.09.2014
(73) Proprietor: Nevro Corporation, Menlo Park, CA 94025 (US)
(72) Inventor: SHARMA, Vivek, San Ramon, CA 94583 (US); CHITRE, Yougandh, Santa Clara, CA 95054 (US); WALKER, Andre, B., Monte Sereno, CA 95030 (US)
(74) Representative: Forresters IP LLP
(86) International application number: PCT/US2012/064092
(87) International publication number: WO 2013/070875

(56) References cited:
- WO-A1-2009/129329
- CN-A- 101 920 065
- US-A- 5 730 628
- US-A1- 2006 264 122
- US-A1- 2009 048 638
- US-A1- 2009 069 803
- US-A1- 2010 256 696
- US-A1- 2010 267 265
- US-B1- 7 299 095

## Description

### TECHNICAL FIELD

The present technology is directed generally to contact assemblies for medical devices, and associated systems and methods. Contact assemblies in accordance with the present technology are suitable for electrical connections between medical device components, including connections between an implantable lead and an implantable pulse generator of a neurological stimulation system.

### BACKGROUND

Neurological stimulators have been developed to treat pain, movement disorders, functional disorders, spasticity, cancer, cardiac disorders, and various other medical conditions. Implantable neurological stimulation systems generally have an implantable pulse generator that is operably coupled to one or more leads that deliver electrical pulses to neurological tissue or muscle tissue. For example, several neurological stimulation systems for spinal cord stimulation (SCS) have cylindrical leads that include a lead body with a circular cross-sectional shape and multiple conductive rings spaced apart from each other at the distal end of the lead body. The conductive rings operate as individual electrodes or contacts to deliver electrical signals to the patient. The SCS leads are typically implanted either surgically or percutaneously through a needle inserted into the epidural space, often with the assistance of a stylet.

Once implanted, the pulse generator applies electrical pulses to the electrodes, which in turn modify the function of the patient's nervous system, such as by altering the patient's responsiveness to sensory stimuli and/or altering the patient's motor-circuit output. In particular, the electrical pulses can generate sensations that mask or otherwise alter the patient's sensation of pain. For example, in many cases, patients report a tingling or paresthesia that is perceived as more pleasant and/or less uncomfortable than the underlying pain sensation. In other cases, the patients can report pain relief without paresthesia or other sensations.

Depending on the treatment location within the patient, lead extensions may be connected between the implantable pulse generator and the lead to provide electrical pulses at more distant locations. Couplings between the pulse generator, the leads, the lead extensions and/or lead adaptors require multiple electrical connections that provide an electrical path to each of the electrodes on a given lead. Each of the electrical connections represents the potential for a fault that can prevent the desired stimulus. Accordingly, the components of the associated connections must be configured to provide a robust electrical connection that reduces the chances of such faults. Additionally, the electrical connections made during a surgical or percutaneous procedure should be simple so as to be coupled and decoupled with low insertion/extraction forces, and yet provide acceptable retention forces. Furthermore, because the connections are implanted in a patient, it is generally necessary for the connections to be compact so as to reduce patient discomfort and/or unsightly bulges at the implant site. Prior systems often include expensive and/or intricate designs to meet the foregoing requirements. Accordingly, there is a need for a low cost contact assembly that provides a robust and/or reliable electrical connection and yet allows a simple, low force coupling/decoupling procedure. Document US2006/264122 discloses a medical device according to the preamble of claim 1.

### BRIEF DESCRIPTION OF THE DRAWINGS

Figure 1 is a partially schematic illustration of an implantable spinal cord modulation system positioned at a patient's spine to deliver therapeutic signals in accordance with an embodiment of the present technology.
Figure 2 is a semi-transparent isometric view of a conductor assembly configured in accordance with a further embodiment of the present technology.
Figure 3 is a semi-transparent isometric view of a portion of a lead extension having a patient implantable element configured in accordance with yet another embodiment of the present technology.
Figure 4 is a semi-transparent isometric view of a portion of an implantable pulse generator having a plurality of receiving elements configured in accordance with still a further embodiment of the present technology.
Figure 5 is an isometric view of a contact assembly having a housing and a contact configured in accordance with another embodiment of the present technology.
Figure 6A is an isometric view of the contact of Figure 5 having a plurality of leaf spring portions in accordance with still another embodiment of the present technology.
Figure 6B is an isometric view of a contact having a plurality of leaf spring portions in accordance with another embodiment of the present technology.
Figure 7 is an isometric view of a contact assembly having a housing with protruding rims configured in accordance with a further embodiment of the present technology.
Figure 8 is a cross-sectional view of the contact assembly of Figure 7.
Figure 9 is a cross-sectional view of a swaged contact assembly configured in accordance with another embodiment of the present technology.
Figure 10 is a cross-sectional view of a contact assembly having a crimped contact configured in accordance with yet another embodiment of the present technology.
Figure 11 is a cross-sectional view of a contact having angled leaf spring portions in accordance with an embodiment of the present technology.

### DETAILED DESCRIPTION

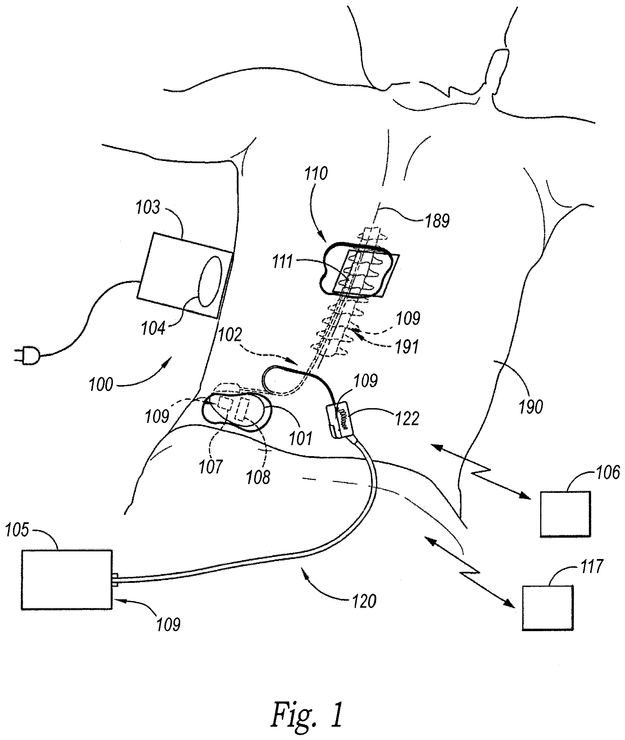

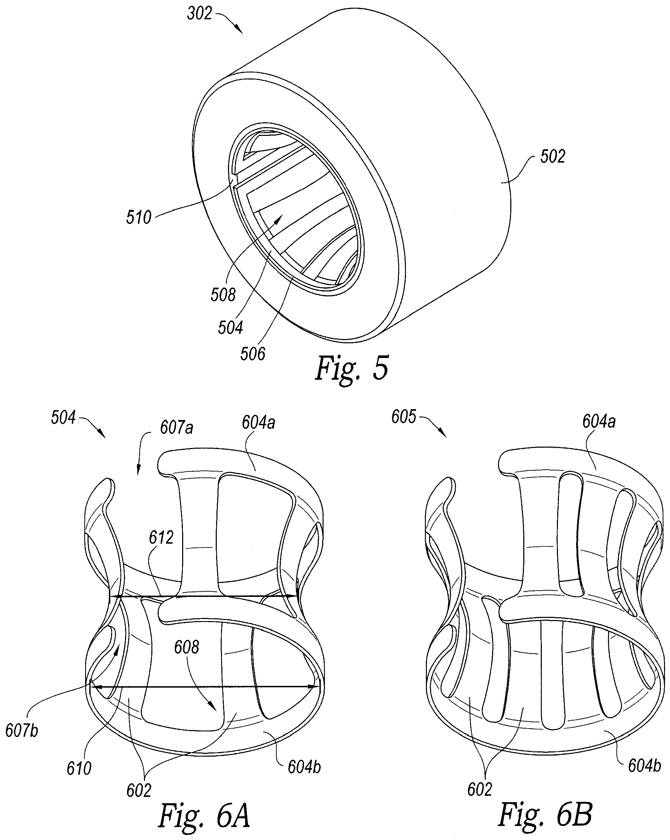

The invention defined by the medical device of independent claim 1. Any disclosed methods are merely exemplary and do not fall within the scope of the present invention. The present technology is directed generally to contact assemblies for medical devices, and more specifically to contact assemblies for implantable neurological stimulation systems. At least some embodiments of the present technology include contact assemblies having housings that carry leaf spring portions. The leaf spring portions can be shaped in various manners (e.g., arcuate, curved, angled) that provide a flexible and secure connection with other device components, including leads and lead extensions. In other embodiments, the devices, systems and associated methods can have different configurations, components, and/or procedures. Still other embodiments may eliminate particular components and/or procedures. A person of ordinary skill in the relevant art, therefore, will understand that the present technology, which includes associated devices, systems, and procedures, may include other embodiments with additional elements or steps, and/or may include other embodiments without several of the features or steps shown and described below with reference to Figures 1-11. Several aspects of overall systems configured in accordance with the disclosed technology are described with reference to Figures 1-4, and features specific to certain contact assemblies are then discussed with reference to Figures 5-11.

Figure 1 schematically illustrates a representative patient system 100 for providing relief from chronic pain and/or other conditions, arranged relative to the general anatomy of a patient's spinal cord 191. The overall patient system 100 can include a signal delivery device 110, which may be implanted within a patient 190, typically at or near the patient's spinal cord midline 189, and coupled to a pulse generator 101. The signal delivery device 110 carries features for delivering therapy to the patient 190 after implantation. The pulse generator 101 can be connected directly to the signal delivery device 110, or it can be coupled to the signal delivery device 110 via a signal link or lead extension 102. In a further representative embodiment, the signal delivery device 110 can include one or more elongated lead(s) or lead body or bodies 111. As used herein, the terms "lead" and "lead body" include any of a number of suitable substrates and/or support members that carry devices for providing therapy signals to the patient 190. For example, the lead or leads 111 can include one or more electrodes or electrical contacts that direct electrical signals into the patient's tissue, such as to provide for patient pain relief. In other embodiments, the signal delivery device 110 can include structures other than a lead body (e.g., a paddle) that also direct electrical signals and/or other types of signals to the patient 190.

The pulse generator 101 can transmit signals (e.g., electrical signals) to the signal delivery device 110 that up-regulate (e.g., stimulate or excite) and/or down-regulate (e.g., block or suppress) target nerves. As used herein, and unless otherwise noted, the terms "modulate" and "modulation" refer generally to signals that have either type of the foregoing effects on the target nerves. The pulse generator 101 can include a machine-readable (e.g., computer-readable) medium containing instructions for generating and transmitting suitable therapy signals. The pulse generator 101 and/or other elements of the system 100 can include one or more processors 107, memories 108 and/or input/output devices. Accordingly, the process of providing modulation signals, providing guidance information for locating the signal delivery device 110, and/or executing other associated functions can be performed by computer-executable instructions contained by computer-readable media located at the pulse generator 101 and/or other system components. The pulse generator 101 can include multiple portions, elements, and/or subsystems (e.g., for directing signals in accordance with multiple signal delivery parameters), carried in a single housing, as shown in Figure 1, or in multiple housings.

In some embodiments, the pulse generator 101 can obtain power to generate the therapy signals from an external power source 103. The external power source 103 can transmit power to the implanted pulse generator 101 using electromagnetic induction (e.g., RF signals). For example, the external power source 103 can include an external coil 104 that communicates with a corresponding internal coil (not shown) within the implantable pulse generator 101. The external power source 103 can be portable for ease of use.

During at least some procedures, an external programmer 105 (e.g., a trial modulator) can be coupled to the signal delivery device 110 during an initial procedure, prior to implanting the pulse generator 101. For example, a practitioner (e.g., a physician and/or a company representative) can use the external programmer 105 to vary the modulation parameters provided to the signal delivery device 110 in real time, and select optimal or particularly efficacious parameters. These parameters can include the location from which the electrical signals are emitted, as well as the characteristics of the electrical signals provided to the signal delivery device 110. In a typical process, the practitioner uses a cable assembly 120 to temporarily connect the external programmer 105 to the signal delivery device 110. The practitioner can test the efficacy of the signal delivery device 110 in an initial position. The practitioner can then disconnect the cable assembly 120 (e.g., at a connector 122), reposition the signal delivery device 110, and reapply the electrical modulation. This process can be performed iteratively until the practitioner obtains the desired position for the signal delivery device 110. Optionally, the practitioner may move the partially implanted signal delivery element 110 without disconnecting the cable assembly 120. Furthermore, in some embodiments, the iterative process of repositioning the signal delivery device 110 and/or varying the modulation parameters, may not be performed.

The pulse generator 101, the lead extension 102, the external programmer 105 and/or the connector 122 can each include a receiving element 109. Accordingly, the receiving elements 109 can be patient implantable elements, or the receiving elements 109 can be integral with an external patient treatment element, device or component (e.g., the external programmer 105 and/or the connector 122). The receiving elements 109 can be configured to facilitate a simple coupling and decoupling procedure between the signal delivery device 110, the lead extension 102, the pulse generator 101, the external programmer 105 and/or the connector 122, as will be described further below.

After a trial period with the external programmer 105, the practitioner can implant the implantable pulse generator 101 within the patient 190 for longer term treatment. The signal delivery parameters provided by the pulse generator 101 can still be updated after the pulse generator 101 is implanted, via a wireless physician's programmer 117 (e.g., a physician's remote) and/or a wireless patient programmer 106 (e.g., a patient remote). Generally, the patient 190 has control over fewer parameters than does the practitioner.

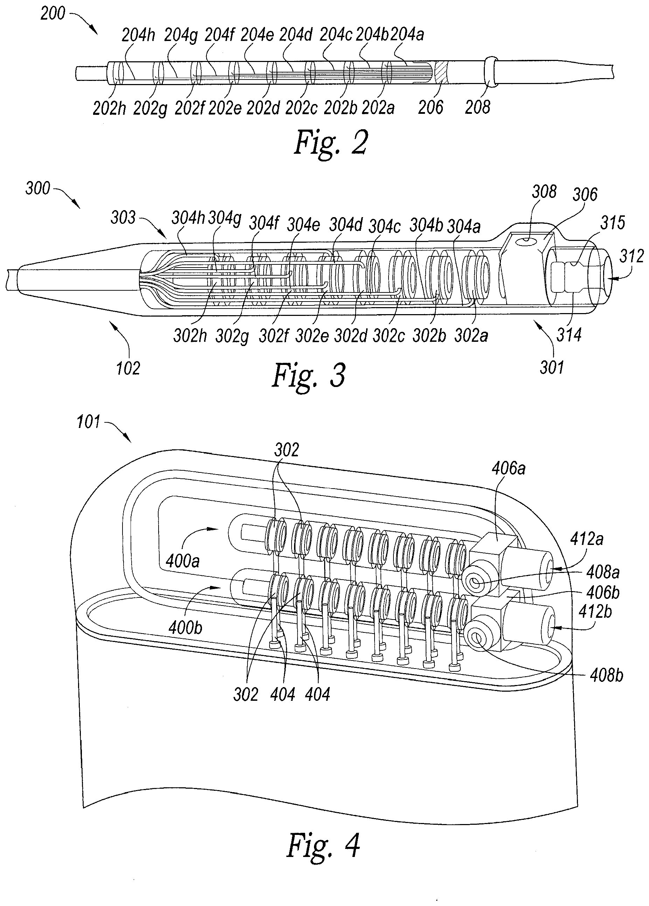

Figure 2 is a semi-transparent isometric view of a conductor assembly 200 configured in accordance with a further embodiment of the present technology. In the illustrated embodiment, the conductor assembly 200 includes multiple ringshaped conductors 202, e.g. eight conductors 202 identified individually as conductors 202a-202h. The conductors 202a-202h are electrically connected to wires 204, identified individually as wires 204a-204h, respectively. The conductor assembly 200 can be positioned at (e.g., integral with) a proximal end of several of the components of the patient system 100 described above with reference to Figure 1. For example, the conductor assembly 200 can be integral with any of the signal delivery devices 110 described above, e.g., the leads 111. In such an embodiment, the wires 204 electrically connect individual conductors 202 to corresponding electrodes carried by the lead 111.

In another embodiment, the conductor assembly 200 can be positioned at (e.g., integral with) a proximal end of the lead extension 102 (Figure 1). In such an embodiment, the wires 204 electrically connect the individual conductors 202 to corresponding contact assemblies carried by a distal end of the lead extension 111, as will be described further below with reference to Figure 3. The conductor assembly 200 further includes a fastening ring 206 and a sealing ring 208. The fastening ring 206 and the sealing ring 208 can engage with features of the receiving elements 109 (Figure 1) to secure and seal a coupling between the conductor assembly 200 and one of the individual receiving elements 109.

As described above with reference to Figure 1, the implantable pulse generator 101, the connector 122 and the lead extension 102 can include receiving elements 109 for connection to the signal delivery device 110 or the lead 111. Figure 3 is a semi-transparent isometric view of a distal end portion of the lead extension 102 having a connector assembly or receiving element (e.g., a patient implantable element 300) configured in accordance with another embodiment of the present technology. In the illustrated embodiment, the patient implantable element 300 includes a passageway or receiving cavity 312 and a plurality of contact assemblies 302 (identified individually as contact assemblies 302a-302h). The contact assemblies 302 can be arranged in a linear manner from proximate a first end 301 of the patient implantable element 300 (e.g., a first end of the receiving cavity 312) to proximate a second end 303 of the patient implantable element 300 (e.g., a second end of the receiving cavity 312). Although the illustrated embodiment includes the contact assemblies 302 arranged linearly, in other embodiments the contact assemblies 302 can be arranged in a curvilinear or nonlinear manner. In the illustrated embodiment, the contact assemblies 302 are molded in place in the patient implantable element 300. In other embodiments, the contact assemblies 302 can be attached to the lead extension 102 by other means. For example, the contact assemblies 302 can be press fit, screwed, strapped or otherwise fastened to the lead extension 102.

The contact assemblies 302a-302h are operably coupled to corresponding wires 304, identified individually as wires 304a-304h, respectively. Although not shown in Figure 3, the proximal end portion of the lead extension 102 can include the conductor assembly 200 of Figure 2. Accordingly, the wires 304 can connect individual contact assemblies 302 to corresponding conductors 202 of the conductor assembly 200. Additionally, although the illustrated embodiment includes the patient implantable element 300 as part of the lead extension 102, in other embodiments, the patient implantable element 300 can be part of (e.g., integral with) other components or devices.

The patient implantable element 300 further includes a securing block 306 having an opening 308 that extends into the receiving cavity 312. The patient implantable element 300 can be configured to receive the conductor assembly 200 (Figure 2) of the lead 111 (Figure 1). Referring to Figures 2 and 3, together, when the conductor assembly 200 is fully inserted into the receiving cavity 312, each of the conductors 202a-202h align with, and are engaged by, a corresponding contact assembly 302a-302h, respectively. A set screw (not shown), or other fastening device (e.g., another leaf spring), can be inserted in the opening 308 to engage the fastening ring 206 (Figure 2) and securely fasten the conductor assembly 200 to the patient implantable element 300. The patient implantable element 300 can encapsulate the wires 304 and the contact assemblies 302 and can include a sealing chamber 314. The sealing chamber 314 can be at least partially defined by a concave surface 315 that extends radially outwardly from the receiving cavity 312. The sealing ring 208 (Figure 2) of the conductor assembly 200 (Figure 2) can be compressively fit within the sealing chamber 314 to form a tight seal and reduce or eliminate the ability of fluids to enter the receiving cavity 312.

Figure 4 is a semi-transparent isometric view of a portion of the implantable pulse generator 101 having multiple receiving elements 400, e.g., a first receiving element 400a and a second receiving element 400b, configured in accordance with still a further embodiment of the present technology. The receiving elements 400 provide for multiple lead extensions 102 or multiple signal delivery devices 110 (e.g., leads 111) to be connected to the implantable pulse generator 101. Similar to the patient implantable element 300, the receiving elements 400 include a plurality of contact assemblies 302 that are operably coupled to conducting wires 404 and can be molded in place or otherwise fastened to the first receiving element 400a or the second receiving element 400b. The first receiving element 400a further includes a first receiving cavity 412a, and a first receiving block 406a having a first opening 408a, each of which functions in a manner generally similar to those described above with reference to Figures 2 and 3. Similar to the first receiving element 400a, the second receiving element 400b includes a second receiving cavity 412b and a second receiving block 406b having a second opening 408b.

As described above, the contact assemblies 302 of Figures 3 and 4 can be configured to engage the conductors 202 of the conductor assembly 200 (Figure 2). Figure 5 is an isometric view of an individual contact assembly 302 having a ringshaped or annular housing 502, a contact 504, and a weld joint 506 configured in accordance with an embodiment of the present technology. The housing 502 further includes an inner surface 510 defining, at least in part, a cylindrical opening 508, which contains the contact 504. The housing 502 can be partially or entirely electrically conductive and the weld joint 506 can operably couple the contact 504 to the housing 502.

Figure 6A is an isometric view of the contact 504 shown in Figure 5. In the illustrated embodiment, the contact 504 is shaped like an open cage, and includes a plurality of semi-elliptical springs or leaf spring portions 602, a first ring portion 604a having a first circumferential opening 607a, and a second ring portion 604b having a second circumferential opening 607b. The leaf spring portions 602 extend from the first ring portion 604a to the second ring portion 604b and bow inwardly or have an inward offset toward the center of the contact 504. The ring portions 604 and the plurality of leaf spring portions 602 define, at least in part, an opening 608, and the arcuate shapes of the bowed leaf spring portions 602 provide an axially varying diameter for the opening 608. For example, the opening 608 can have a first diameter 610 at the ring portions 604 and a second diameter 612 at a midpoint of the leaf spring portions 602 between the ring portions 604. Although the illustrated embodiments include the housing 502 and the contact 504 having circular openings 508 and 608, respectively, in other embodiments, the openings 508 and 608 can be in other shapes, e.g., elliptical.

The illustrated embodiment of the contact 504 includes six individual leaf spring portions 602. In other embodiments, contacts can include additional or fewer leaf spring portions 602. For example, Figure 6B is an isometric view of a contact 605 that includes ten individual leaf spring portions 602. By properly selecting the number and/or the width of the leaf spring portions 602, contacts can be made to have different compressive tendencies that provide corresponding different individual coupling and decoupling properties.

The contact 504 can be fabricated (e.g., by casting, stamping or other suitable processes) from a variety of metals or metal alloys. For example, in some embodiments, the contact 504 includes MP35N, stainless steel, titanium and/or a platinum/iridium alloy such as 80/20 or 90/10 Pt/lr. Similarly, the housing 502 can also be constructed of metals or metal alloys, including MP35N, stainless steel and/or titanium. Additionally, the contact 504 and the housing 502 can each be formed from one continuous piece of metal that is cast or fabricated into the finished form. For example, fabrication methods for the contact 504 and/or the housing 502 can include the use of a computer numerical control (CNC) machine to shape stock metal or metal alloys. Additionally, the contact 504 can be formed from a piece of metal in a multi-step process. First, the piece of metal can be stamped to form a blank. Portions of the blank can then be removed to form a cage that can be roll-bended to form a ring cage. The ring cage can then be bent to form a series of leaf springs and heat treated to impart desired characteristics to the finished contact. In other embodiments, the multi-step process can include less than all of the foregoing steps, e.g., any suitable combination of the foregoing steps.

Referring to Figures 2, 5, and 6A together, the conductors 202 (Figure 2) can be configured to have a diameter smaller than the first diameter 610 and larger than the second diameter 612 of the opening 608 (Figure 6A). In operation, an individual conductor 202 of the conductor assembly 200 can be inserted into the opening 608. The individual conductor 202 proceeds into the opening 608 and engages the leaf spring portions 602 (Figure 6A), flexing the leaf spring portions 602 outwardly. Flexing or deforming (e.g., plastically deforming) the leaf spring portions 602 creates a compressive force that keeps the leaf spring portions 602 firmly pressed against or engaged with the individual conductor 202. The firm physical contact between the conductor 202 and the leaf spring portions 602 creates a robust mechanical and electrical connection between the conductor 202 and the metal contact assembly 302 (Figure 5). Accordingly, electrical current can reliably flow from the housing 502 (Figure 5) to the contact 504 (Figures 5 and 6), and to the conductor 202.

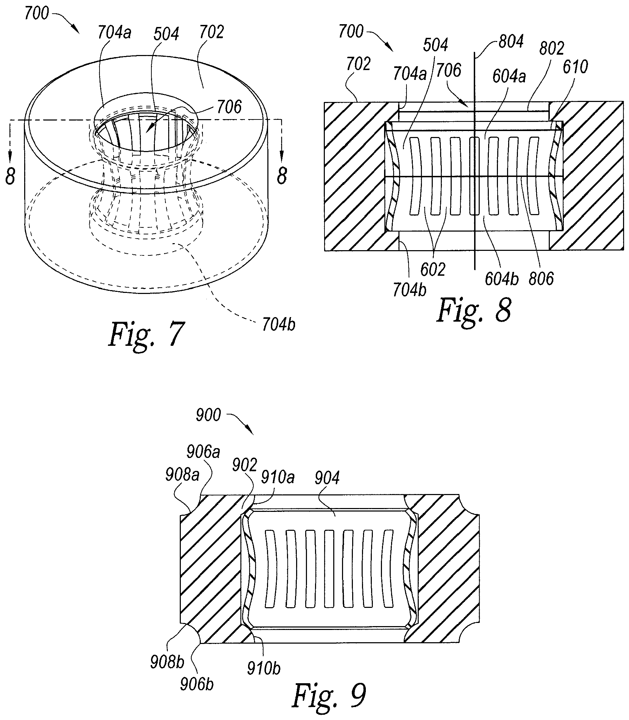

Although the contact assembly 302 of Figure 5 includes a weld joint 506 between the contact 504 and the housing 502, other embodiments can have other arrangements for coupling the contact to the housing. Figure 7 is an isometric view of a contact assembly 700 having a housing 702 with inwardly protruding rims 704 (identified individually as a first protruding rim 704a and a second protruding rim 704b) configured in accordance with a further embodiment of the present technology. The protruding rims 704 extend toward the center of an opening 706 in the housing 702 and can contain, capture or hold the contact 504 within the housing. Figure 8 is a cross-sectional view of the contact assembly 700 along the line 8-8 of Figure 7. The contact 504 can be compressed, making the diameter 610 at the rings 604 smaller than a diameter 802 of the opening 706 at the protruding rims 704. In this manner, the contact 504 can be inserted into the housing 702 from either above or below. For example, while compressed, the contact 504 can be pushed into the opening 706 from above until both rings 604 are past the protruding rim 704a. The contact 504 subsequently expands, such that the diameter 610 at the rings 604 is greater than the diameter 802 at the protruding rims 704. The contact 504 is thereby contained within the housing 702. Additionally, the housing 702 can have an interior diameter 806 that is smaller than the diameter 610 at the rings 604 when the contact 504 is in a "relaxed" state. In this manner, the housing 702 can keep the contact 504 slightly compressed to help maintain a robust physical coupling between the contact 504 and the housing 702.

In the illustrated embodiment, the contact 504 abuts the protruding rims 704. When a conductor 202 (Figure 2) is inserted into the contact assembly 700, the leaf spring portions 602 flex outwardly. Flexing the leaf spring portions 602 causes the contact 504 to exert pressure against the protruding rims 704. The protruding rims 704 hold the contact 504 in place, increasing the compression in the leaf spring portions 602, and further securing the contact 504 in the housing 702. In other embodiments, a gap can be present between the contact 504 and one or more of the protruding rims 704. In such an embodiment, inserting a conductor 202 into the contact assembly 702 can expand the contact 504 along an insertion axis 804, in addition to flexing of the leaf spring portions 602. Even with room for such an axial expansion, the contact 504 maintains a snug fit with the housing 702 through the radial compressive force in the leaf spring portions 602. Accordingly, the contact 504 can be designed to provide for secure contact with an inserted conductor 202 with or without expanding the contact 504 along the insertion axis 804.

In a further embodiment, a contact assembly can be swaged or compressed to provide a secure connection between the contact and the housing. Figure 9 is a cross-sectional view of a contact assembly 900 configured in accordance with another embodiment of the technology. The contact assembly 900 includes a housing 902 and a contact 904. The housing 902 can be swaged along a first circumference 906a and a second circumference 906b creating a first exterior deformation 908a and a second exterior deformation 908b, respectively. The swaging also creates a first interior deformation 910a and a second interior deformation 910b. The first interior deformation 910a and the second interior deformation 910b can compress and/or otherwise engage the contact 904, securing the contact 904 within the housing 902 and creating a solid coupling between the contact 904 and the housing 902.

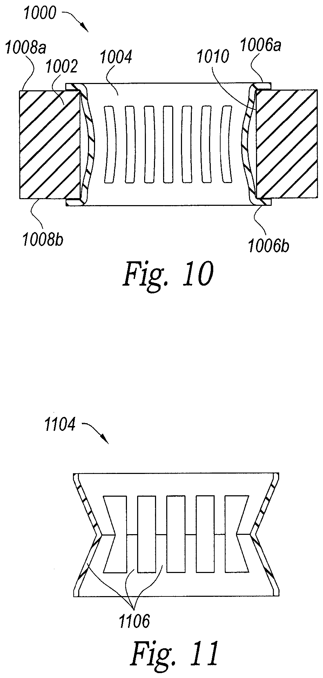

Contact assemblies in accordance with other embodiments can include components that are crimped to secure and couple the contact to the housing. Figure 10 is a cross-sectional view of a contact assembly 1000 having a crimped contact 1004 secured to a housing 1002. A folded first ring 1006a and a folded second ring 1006b can be formed by crimping or bending the contact 1004. In the illustrated embodiment, the contact 1004 engages the housing 1002 along a first surface 1008a, a second surface 1008b, and an interior surface 1010. In other embodiments, the contact 1004 can be constructed and crimped to engage more or less of the housing 1002. For example, the contact 1004 can be constructed and crimped to only engage the housing 1002 along the first and second surfaces 1008a and 1008b. In still other embodiments, the contact 1004 and/or the housing 1002 can be crimped in other manners to couple the housing 1002 and the contact 1004.

In still another embodiment, a contact can include angular leaf spring portions. Figure 11 is a cross-sectional view of a contact 1104 configured in accordance with another embodiment of the present technology. Similar to the contacts 504 and 605 of Figures 6A and 6B, the contact 1104 includes a plurality of leaf spring portions 1106. However, the leaf spring portions 1106 are angled inwardly, rather than having a curved or arcuate shape. The angled leaf spring portions 1106 function in a manner generally similar to that described above with reference to the leaf spring portions 602 of Figures 6A and 6B. Accordingly, contacts in accordance with the present technology can include leaf spring portions having a variety of shapes and configurations.

From the foregoing, it will be appreciated that specific embodiments of the disclosed technology have been described herein for purposes of illustration, but that various modifications may be made without deviating from the technology. For example, rather than rings or cages having circumferential openings, the contacts can be closed rings or cages. Other materials may be used in place of those described herein, or additional components may be added or removed. For example, although the illustrated embodiments include six or ten leaf spring portions that are equally spaced circumferentially around a contact, other embodiments may use fewer or additional leaf spring portions, or a different pattern. Furthermore, although the illustrated embodiments include patient implantable elements, receiving elements, and conductor assemblies having eight individual contact assemblies or conductors, other embodiments may include more or less contact assemblies or conductors.

Contact assemblies having contacts and/or housings in accordance with the present technology may be configured to be coupled by methods in addition to those described above. Such methods can include soldering, brazing, and/or other coupling methods.

## Claims

1. An electrically powered medical device, comprising:
a patient implantable element having a receiving cavity; and
at least one contact assembly positioned in the receiving cavity, the contact assembly comprising:
a housing having an annular shape with an inner surface defining at least in part an opening; and
a contact (504) disposed at least partially within the opening, the contact having:
a first ring portion (604a) and a second ring portion (604b),
a plurality of leaf spring portions (602), wherein the plurality of leaf spring portions (602) extend between the first ring portion (604a) and the second ring portion (604b), and **characterised in that** the first ring portion (604a) and the second ring portion (604b) include circumferential openings (607a, 607b).

2. The electrically powered medical device of claim 1 wherein the first ring portion, the second ring portion and the plurality of leaf spring portions comprise one continuous piece of metal.

3. The electrically powered medical device of claim 1 wherein at least one of the first ring portion and the second ring portion is welded to the housing.

4. The electrically powered medical device of claim 1 wherein the housing and the contact are joined by a crimped joint.

5. The electrically powered medical device of claim 1 wherein the housing and the contact are joined by a swaged joint.

6. The electrically powered medical device of claim 1 wherein the housing further includes at least one protruding rim that extends inwardly into the opening, and wherein the contact is press fit into the opening and the rim at least partially secures the contact.

7. The electrically powered medical device of claim 1, further comprising a conducting wire operably coupled to the housing.

8. The electrically powered medical device of claim 1, further comprising an implantable pulse generator, and wherein the patient implantable element is carried by the implantable pulse generator.

9. The electrically powered medical device of claim 1, wherein
a metal cage is disposed at least partially within the opening, the metal cage comprising: the first ring portion; the second ring portion; and the plurality of leaf spring portions extending from the first ring portion to the second ring portion and the individual spring portions have an inward offset.

10. The electrically powered medical device of claim 9 wherein the spring portions have a curved shape and the curved shape at least partially defines the inward offset of the spring portions.

11. The electrically powered medical device of claim 9 wherein the spring portions have an angular shape and the angular shape at least partially defines the inward offset of the spring portions.

12. The electrically powered medical device of claim 1, wherein the at least one contact assembly comprises a plurality of contact assemblies disposed sequentially from proximate a first end of the receiving cavity to proximate a second end of the receiving cavity, and
the device further comprising a plurality of conducting wires, individual conducting wires operably coupled to corresponding individual contact assemblies.

13. The electrically powered medical device of claim 12, further comprising a sealing chamber proximate the first end of the receiving cavity, the sealing chamber including a concave surface positioned radially outwardly from the receiving cavity.

14. The electrically powered medical device of claim 12, further comprising a securing block proximate the first end of the receiving cavity, the securing block having an opening extending into the receiving cavity.

## Patentansprüche

1. Elektrisch angetriebene medizinische Vorrichtung, umfassend:
ein in einen Patienten implantierbares Element, das einen Aufnahmehohlraum aufweist; und mindestens eine in dem Aufnahmehohlraum positionierte Kontaktanordnung, wobei die Kontaktanordnung umfasst:
ein Gehäuse aufweisend eine Ringform mit einer Innenfläche, die mindestens zum Teil eine Öffnung definiert; und
einen Kontakt (504), der mindestens teilweise innerhalb der Öffnung angeordnet ist, wobei der Kontakt aufweist:
einen ersten Ringabschnitt (604a) und einen zweiten Ringabschnitt (604b) und
eine Vielzahl von Blattfederabschnitten (602), wobei sich die Vielzahl von Blattfederabschnitten (602) zwischen dem ersten Ringabschnitt (604a) und dem zweiten Ringabschnitt (604b) erstreckt, und **dadurch gekennzeichnet, dass** der erste Ringabschnitt (604a) und der zweite Ringabschnitt (604b) Umfangsöffnungen (607a, 607b) beinhalten.

2. Elektrisch angetriebene medizinische Vorrichtung nach Anspruch 1, wobei der erste Ringabschnitt, der zweite Ringabschnitt und die Vielzahl von Blattfederabschnitten ein durchgehendes Stück Metall umfassen.

3. Elektrisch angetriebene medizinische Vorrichtung nach Anspruch 1, wobei mindestens einer des ersten Ringabschnitts und des zweiten Ringabschnitts an das Gehäuse geschweißt ist.

4. Elektrisch angetriebene medizinische Vorrichtung nach Anspruch 1, wobei das Gehäuse und der Kontakt durch eine Quetschverbindung gefügt sind.

5. Elektrisch angetriebene medizinische Vorrichtung nach Anspruch 1, wobei das Gehäuse und der Kontakt durch eine Pressverbindung gefügt sind.

6. Elektrisch angetriebene medizinische Vorrichtung nach Anspruch 1, wobei das Gehäuse ferner mindestens einen vorstehenden Rand beinhaltet, der sich in die Öffnung nach innen erstreckt, und wobei der Kontakt in die Öffnung pressgepasst wird und der Rand mindestens teilweise den Kontakt sichert.

7. Elektrisch angetriebene medizinische Vorrichtung nach Anspruch 1, ferner umfassend einen leitenden Draht in Wirkverbindung mit dem Gehäuse.

8. Elektrisch angetriebene medizinische Vorrichtung nach Anspruch 1, ferner umfassend einen implantierbaren Impulsgenerator, und wobei das in einen Patienten implantierbare Element von dem implantierbaren Impulsgenerator getragen wird.

9. Elektrisch angetriebene medizinische Vorrichtung nach Anspruch 1, wobei ein Metallkäfig mindestens teilweise innerhalb der Öffnung angeordnet ist, wobei der Metallkäfig umfasst: den ersten Ringabschnitt; den zweiten Ringabschnitt; und die Vielzahl von Blattfederabschnitten, die sich von dem ersten Ringabschnitt zu dem zweiten Ringabschnitt erstrecken, und die individuellen Federabschnitte einen Einwärtsversatz aufweisen.

10. Elektrisch angetriebene medizinische Vorrichtung nach Anspruch 9, wobei die Federabschnitte eine gekrümmte Form aufweisen und die gekrümmte Form mindestens teilweise den Einwärtsversatz der Federabschnitte definiert.

11. Elektrisch angetriebene medizinische Vorrichtung nach Anspruch 9, wobei die Federabschnitte eine Winkelform aufweisen und die Winkelform mindestens teilweise den Einwärtsversatz der Federabschnitte definiert.

12. Elektrisch angetriebene medizinische Vorrichtung nach Anspruch 1, wobei die mindestens eine Kontaktanordnung eine Vielzahl von Kontaktanordnungen umfasst, die sequenziell von nahe einem ersten Ende des Aufnahmehohlraums zu nahe einem zweiten Ende des Aufnahmehohlraums angeordnet sind, und
die Vorrichtung ferner eine Vielzahl von leitenden Drähten umfasst, wobei individuelle leitende Drähte in Wirkverbindung mit entsprechenden individuellen Kontaktanordnungen sind.

13. Elektrisch angetriebene medizinische Vorrichtung nach Anspruch 12, ferner umfassend eine Abdichtkammer nahe dem ersten Ende des Aufnahmehohlraums, wobei die Abdichtkammer eine konkave Oberfläche beinhaltet, die radial nach außen in Bezug auf den Aufnahmehohlraum positioniert ist.

14. Elektrisch angetriebene medizinische Vorrichtung nach Anspruch 12, ferner umfassend einen Sicherungsblock nahe dem ersten Ende des Aufnahmehohlraums, wobei der Sicherungsblock eine sich in den Aufnahmehohlraum erstreckende Öffnung aufweist.

## Revendications

1. Dispositif médical électrique, comprenant :
un élément implantable chez un patient, ayant une cavité de réception ; et
au moins un ensemble contact positionné dans la cavité de réception, l'ensemble contact comprenant :
un boîtier ayant une forme annulaire avec une surface interne définissant, au moins en partie, une ouverture ; et
un contact (504) disposé au moins en partie dans l'ouverture, le contact ayant :
une première partie anneau (604a) et une seconde partie anneau (604b), et
une pluralité de parties ressorts à lame (602), la pluralité de parties ressorts à lame (602) s'étendant entre la première partie anneau (604a) et la seconde partie anneau (604b), le dispositif médical électrique étant **caractérisé en ce que** la première partie anneau (604a) et la seconde partie anneau (604b) incluent des ouvertures circonférentielles (607a, 607b).

2. Dispositif médical électrique selon la revendication 1, dans lequel la première partie anneau, la seconde partie anneau et la pluralité de parties ressorts à lame comprennent une pièce de métal continue.

3. Dispositif médical électrique selon la revendication 1, dans lequel la première partie anneau et/ou la seconde partie anneau sont soudées au boîtier.

4. Dispositif médical électrique selon la revendication 1, dans lequel le boîtier et le contact sont joints par un raccord poinçonné.

5. Dispositif médical électrique selon la revendication 1, dans lequel le boîtier et le contact sont joints par un raccord embouti.

6. Dispositif médical électrique selon la revendication 1, dans lequel le boîtier inclut en outre au moins un rebord en saillie qui s'étend vers l'intérieur dans l'ouverture, et dans lequel le contact est ajusté avec serrage dans l'ouverture et le rebord fixe au moins en partie le contact.

7. Dispositif médical électrique selon la revendication 1, comprenant en outre un fil conducteur couplé de manière fonctionnelle au boîtier.

8. Dispositif médical électrique selon la revendication 1, comprenant en outre un générateur d'impulsions implantable, l'élément implantable chez un patient étant porté par le générateur d'impulsions implantable.

9. Dispositif médical électrique selon la revendication 1, dans lequel une cage métallique est disposée au moins en partie dans l'ouverture, la cage métallique comprenant : la première partie anneau ; la seconde partie anneau ; et la pluralité de parties ressorts à lame s'étendant de la première partie anneau à la seconde partie anneau, les parties ressorts individuelles ayant un décalage vers l'intérieur.

10. Dispositif médical électrique selon la revendication 9, dans lequel les parties ressorts ont une forme incurvée et la forme incurvée définit au moins en partie le décalage vers l'intérieur des parties ressorts.

11. Dispositif médical électrique selon la revendication 9, dans lequel les parties ressorts ont une forme angulaire et la forme angulaire définit au moins en partie le décalage vers l'intérieur des parties ressorts.

12. Dispositif médical électrique selon la revendication 1, dans lequel l'au moins un ensemble contact comprend une pluralité d'ensembles contacts disposés séquentiellement d'une proximité d'une première extrémité de la cavité de réception à une proximité d'une seconde extrémité de la cavité de réception, et
le dispositif comprenant en outre une pluralité de fils conducteurs, des fils conducteurs individuels étant couplés de manière fonctionnelle à des ensembles contacts individuels correspondants.

13. Dispositif médical électrique selon la revendication 12, comprenant en outre une chambre étanche à proximité de la première extrémité de la cavité de réception, la chambre étanche incluant une surface concave positionnée radialement vers l'extérieur de la cavité de réception.

14. Dispositif médical électrique selon la revendication 12, comprenant en outre un bloc de fixation à proximité de la première extrémité de la cavité de réception, le bloc de fixation ayant une ouverture s'étendant dans la cavité de réception.
